# EUROPÄISCHE PATENTSCHRIFT

(11) **EP 1 656 903 B1**
(45) Veröffentlichungstag und Bekanntmachung des Hinweises auf die Patenterteilung: **21.05.2008**
(21) Anmeldenummer: 05024679.2
(22) Anmeldetag: 11.11.2005
(51) Int. Cl.: A61C 1/05, A61C 17/02

(54) **Medizinisches, insbesondere dentalmedizinisches, längliches Handstück mit einer Haltevorrichtung für ein Werkzeug**
Elongated medical in particular dental handpiece with a support device for a tool
Pièce à main médicale, en particulier dentaire allongée comportant un dispositif de maintien d'un outil

(30) Priorität: 12.11.2004 DE 202004017597 U
(43) Veröffentlichungstag der Anmeldung: 17.05.2006
(73) Patentinhaber: Kaltenbach & Voigt GmbH, 88400 Biberach (DE)
(72) Erfinder: Kuhn, Bernhard, 88400 Biberach (DE)
(74) Vertreter: Schmidt-Evers, Jürgen

(56) Entgegenhaltungen:
- DE-U1- 29 917 857
- US-A- 2 685 737
- US-A- 3 815 241
- US-A- 4 017 974

## Beschreibung

Die Erfindung betrifft ein medizinisches Handstück nach dem Oberbegriff des Anspruchs 1.

Es ist der Zweck eines solchen Handstücks, mit einem im vorderen Endbereich des länglichen Handstücks mittels einer Haltevorrichtung gehaltenen Werkzeugs mittelbar oder unmittelbar auf eine Behandlungsstelle am menschlichen oder tierischen Körper oder an einer Prothese einzuwirken. Dabei sind in vielen Fällen besondere Anforderungen an die Zugänglichkeit zur Behandlungsstelle, insbesondere in Körperhöhlen, zu erfüllen, und deshalb ist für ein Handstück der vorliegenden Art eine beschränkte Baugröße und auch Bauform vorgegeben. Insbesondere dann, wenn eine vom Handstück seitlich gelegene Behandlungsstelle bearbeitet werden soll, eignet sich ein sogenanntes Winkel-Handstück, das eine seitlich angeordnete und seitlich zugängliche Haltevorrichtung für ein Werkzeug aufweist. Eine solche Bauart eignet sich nicht nur für eine seitliche Bearbeitung sondern auch für eine Bearbeitung in einer Körperhöhle des zu behandelnden Körpers, z.B. im Mundraum eines Patienten.

Bekannte Winkel-Handstücke weisen eine Auslassdüse für ein strömendes Medium auf, mit der ein Medienstrahl abgebbar ist, der in einer die Auslassdüse enthaltenden Längsmittelebene verläuft.

Aus der US 2 685 737 ist ein medizinisches Handstück nach dem Oberbegriff des Anspruchs 1 bekannt.

Der Erfindung liegt die Aufgabe zugrunde, ein Handstück der eingangs angegebenen Art so auszugestalten, dass es sich auch für Werkzeuge unterschiedlicher Größe und/oder Formen eignet.

Diese Aufgabe wird durch die Merkmale des Anspruchs 1 gelöst. Vorteilhafte Weiterbildungen der Erfindung sind in den Unteransprüchen beschrieben.

Der Erfindung liegt die Erkenntnis zugrunde, dass bei der Benutzung von Werkzeugen unterschiedlicher Größen und/oder Formen sich Behandlungsstellen ergeben können, die bezüglich einer Haltevorrichtung für das Werkzeug in unterschiedlichen Abständen und/oder Winkelpositionen angeordnet sein können, in denen die Beaufschlagung mit dem Medium unzureichend ist.

Bei der erfindungsgemäßen Ausgestaltung ist die Auslassdüse zwecks Einstellung der Richtung des Medienstrahles verstellbar. Hierdurch ist es möglich, das Handstück durch ein Verstellen der Auslassdüse auf eine gewünschte Behandlungsstelle einzurichten, diese gezielt mit dem Medium zu beaufschlagen und dadurch zum einen die Anpassbarkeit des Handstücks an unterschiedliche Bearbeitungs- oder Behandlungsstellen zu verbessern und zum anderen eine gezielte Mediumzuführung zu der gewünschten Behandlungsstelle zu ermöglichen.

Im Rahmen der Erfindung gibt es viele Möglichkeiten, die Auslassdüse so verstellbar auszubilden, dass sie zwecks Einstellung der Richtung des Medienstrahles verstellbar ist. Dabei ist es vorteilhaft, die Auslassdüse auf ihren Verstellweg in einer Führung zu führen, so dass die Bewegungsrichtung definiert ist.

Es ist im weiteren vorteilhaft, die Auslassdüse aus einer Mittelstellung heraus in zwei einander etwa entgegengesetzt gerichtete Bewegungsrichtungen verstellbar anzuordnen. Hierdurch eignet sich das Handstück sowohl als Rechtsausführung als auch als Linksausführung. Außerdem wird hierdurch der Einstellbereich vergrößert und die Anpassbarkeit des Handstücks an unterschiedliche Behandlungsstellen verbessert.

Eine vorteilhafte Bewegung für die Auslassdüse wird auch dann erreicht, wenn diese hin und her schwenkbar gelagert ist.

Insbesondere bei solchen Handstücken, bei denen die Auslassdüse einen nach hinten gerichteten Abstand von der Haltevorrichtung für das Werkzeug aufweist, ist es erfindungsgemäß, die Auslassdüse in der Umfangsrichtung des Handstücks bewegbar, insbesondere schwenkbar, anzuordnen. Dabei kann die Auslassdüse in vorteilhafter Weise an einer Drehhülse angeordnet sein, die sich am Umfang des Handstücks befindet und darauf drehbar gelagert ist, vorzugsweise wenigstens teilweise in das Handstück versenkt angeordnet ist. Dabei kann die Drehhülse auf einem sie tragenden Drehlagerzapfen drehbar gelagert sein.

Um dabei zum einen eine einfache und zum anderen eine dichte Ausgestaltung zu gewährleisten, ist es vorteilhaft, einen sich zur Auslassdüse erstreckenden Zuführungs-Leitungsabschnitt als eine zwischen der Drehhülse und dem Drehlagerzapfen vorhandene Ringnut auszubilden und zu beiden Seiten der Ringnut eine Ringdichtung zwischen der Drehhülse und dem Drehlagerzapfen auszubilden. Eine solche Ringdichtung ist vorzugsweise durch einen Dichtring, z.B. ein O-Ring, gebildet, der in einer Ringnut sitzt, die vorzugsweise in der Mantelfläche des Drehlagerzapfens angeordnet ist.

Es ist zur Vereinfachung der Handhabung im weiteren vorteilhaft, den Verstellweg der Auslassdüse durch wenigstens einen Anschlag zu begrenzen. Hierzu kann ein radialer Anschlagzapfen dienen, der sich in ein in die Umfangsrichtung des Handstücks gerichtetes Langloch erstreckt.

Die Erfindung eignet sich insbesondere für ein solches Handstück, das eine Haltevorrichtung für das Werkzeug aufweist, wobei zwischen der Haltevorrichtung und dem Werkzeug eine Drehpositioniervorrichtung angeordnet ist, so dass das Werkzeug in wenigstens zwei oder mehr in die Umfangsrichtung zueinander versetzten Stellungen positionierbar ist. Bei einem solchen Handstück lässt sich die Auslassdüse auf bestimmte Stellen, z.B. am Umfang des Werkzeugs, richten. Dabei kann ein Arbeitsabschnitt des Werkzeugs exzentrisch angeordnet sein, z.B. in Form einer Segmentscheibe.

Bei allen diesen Ausführungsbeispielen ermöglicht die erfindungsgemäße Verstellbarkeit der Auslassdüse deren Anpassung an gewünschte Abstrahlrichtungen. Aber auch grundsätzlich ermöglicht die erfindungsgemäße Verstellung der Auslassdüse eine wahlweise Anpassung des Handstücks an eine Rechts/Linksausführung für Rechts- oder Linkshänder.

Weitere Weiterbildungsmerkmale der Erfindung ermöglichen eine kleine und kompakte Konstruktion des Handstücks bei Gewährleistung einer guten und sicheren Funktion auch unter Berücksichtigung eines sich durch das Handstück erstreckenden Bewegungsantriebs für das Werkzeug, insbesondere eines Antriebs für eine Hin- und Her-Bewegung des Werkzeugs bzw. der Haltevorrichtung um eine Schwenkachse.

Nachfolgend wird die Erfindung anhand von vorteilhaften Ausgestaltungen eines Ausführungsbeispiels näher erläutert. Es zeigt:
- Fig. 1: ein medizinisches oder dentalmedizinisches Behandlungsinstrument mit *einem länglichen Handstück in der Seitenansicht;*
- Fig. 2: den mit X gekennzeichneten vorderen Endbereich des Handstücks im vertikalen Längsschnitt und in vergrößerter Darstellung;
- Fig. 3: den vorderen Endbereich des Handstücks in der axialen Rückansicht gemäß dem Pfeil V in Fig. 2, wobei eine Auslassdüse des Handstücks sich in einer Bewegungsendstellung befindet,
- Fig. 4: den vorderen Endbereich in der Ansicht gemäß Fig. 3, wobei sich die Auslassdüse in einer gegenüberliegenden Bewegungsendstellung befindet;
- Fig. 5: den Schnitt V-V in Fig. 2 in vergrößerter Darstellung;
- Fig. 6: ein Halteelement eines Werkzeugs für das Handstück im axialen Schnitt.

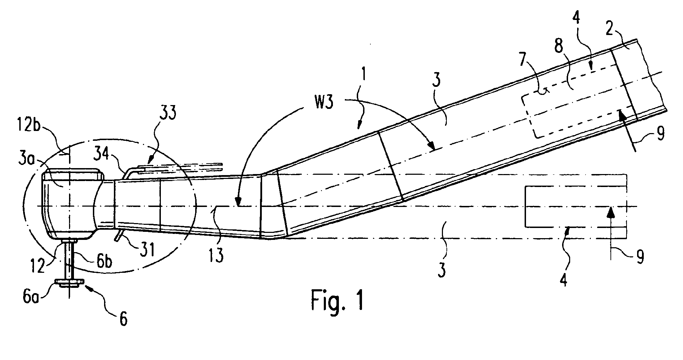

Das in Fig. 1 in seiner Gesamtheit mit 1 bezeichnete Behandlungsinstrument besteht aus einem hinteren Instrumententeil nämlich einem sog. Anschlussteil 2, und einem vorderen Instrumententeil, nämlich einem sog. Handstück 3, die durch eine Steckkupplung 4, insbesondere eine Steck/Drehkupplung, lösbar miteinander verbunden sind. Im vorderen Endbereich des Handstücks 3 ist eine vorzugsweise lösbare Haltevorrichtung 5 für ein Werkzeug 6 angeordnet, die das Werkzeug 6 in einer seitlich abstehenden Position hält.

Das Handstück kann sich gerade oder winkelförmig erstrecken wie es in Fig. 1 strichpunktiert und mit durchgezogenen Linien dargestellt ist. Diese beiden Handstück-Ausgestaltungen werden in der Fachsprache jeweils mit "Winkelhandstück" bezeichnet, was weiter unten noch erklärt wird.

Die Steck/Drehkupplung wird durch eine im Querschnitt runde Kupplungsausnehmung 7 und einen darin mit geringem Bewegungsspiel einsteckbaren Kupplungszapfen 8 gebildet. Beim vorliegenden Ausführungsbeispiel ist die Kupplungsausnehmung 7 am hinteren Ende des Handstücks 3 angeordnet, und der im wesentlichen zylindrische oder stufenzylindrische Kupplungszapfen 8 erstreckt sich vom Anschlussteil 2 nach vorne. Im gekuppelten Zustand sind die Kupplungsausnehmung 7 und der Kupplungszapfen 8 durch eine Verrastungsvorrichtung mit einem andeutungsweise dargestellten Verrastungselement 9 lösbar miteinander verrastet, das in dem einen Kupplungsteil radial beweglich gelagert und durch eine Federkraft in eine die Teilungsfuge zwischen den Kupplungsteilen durchsetzende Verrastungsstellung vorgespannt ist, in der das Verrastungselement 9 in eine Ringnut im anderen Kupplungsteil einfasst. Eine solche an sich bekannte Verrastungsvorrichtung ist durch eine manuelle Zugkraftausübung zum Trennen der Kupplungsteile überdrückbar, wobei das Verrastungselement 9 selbsttätig in seine Freigabestellung verdrängt wird.

Das Anschlussteil 2 ist in nicht dargestellter Weise mit einer flexiblen Versorgungsleitung verbindbar oder verbunden, die mit einem Steuergerät verbunden ist. Das Handstück 3 ist beim Vorhandensein der Steck/Drehkupplung um 360° frei drehbar am Anschlussteil 2 gelagert, wodurch die Handhabbarkeit des Handstücks 3 verbessert wird.

Die Haltevorrichtung 5 weist eine Aufnahmehülse 12 zur Aufnahme des Werkzeugs 6 auf, deren eines Ende eine von der Werkzeugseite her zugängliche Einstecköffnung 12a für das Werkzeug 6 bzw. dessen Halteelement hat. Die Aufnahmehülse 12 ist in einer sich quer, z.B. rechtwinklig, zur Längsachse 13 des Handstücks 3 erstreckenden Position in einer entsprechenden Lageranordnung im vorderen Endbereich des Handstücks 3 bewegbar gelagert. Beim vorliegenden Ausführungsbeispiel ist eine hin und her gehende Schwenkbewegung um die Längsmittelachse 12b der Aufnahmehülse 12 vorgesehen. Zur Lagerung sind zwei Drehlager 14a, 14b, z.B. Wälzlager, zwischen endseitigen Lagerzapfen 15a, 15b und der Innenumfangswand einer Lagerbohrung 16 angeordnet.

Die Aufnahmehülse 12 bzw. die Haltevorrichtung 5 lässt sich durch einen Antrieb 17 mit einem nicht dargestellten Antriebsmotor bewegen, der im oder außerhalb des Handstücks 3 angeordnet sein kann, z.B. im Anschlussteil 2. Beim Ausführungsbeispiel erstreckt sich längs durch das Handstück 3 bzw. Instrument 1 eine Antriebswelle 18, die mit der Aufnahmehülse 12 bzw. der Haltevorrichtung 5 in Antriebsverbindung steht. Beim Ausführungsbeispiel ist die Antriebsverbindung durch eine Kulissenführung 19 und einen darin einfassenden Excenterzapfen 21 gebildet, die zwischen der Antriebswelle 18 und der Haltevorrichtung 5 bzw. der Aufnahmehülse 12 wirksam sind. Beim Ausführungsbeispiel ist der vorzugsweise kugelförmig gerundete Excenterzapfen 21 an der Antriebswelle 18 angeordnet, und er fasst mit geringem Bewegungsspiel in eine axiale Kulissennut 19a der Haltevorrichtung 5 bzw. der Aufnahmehülse 12 ein. Hierdurch wird im wahlweise einschaltbaren Drehbetrieb die Haltevorrichtung 5 die Aufnahmehülse 12 in der Umfangsrichtung hin und her geschwenkt.

Das Werkzeug 6 kann mit einem Arbeitsabschnitt 6a und einem von diesen abstehenden Halteelement, z.B. einem im wesentlichen zylindrischen Halteschaft 6b, einteilig oder zweiteilig ausgebildet sein.

Der Halteschaft 6b ist durch eine Kupplungsanordnung mit der Haltevorrichtung 5 bzw. der Aufnahmehülse 2 verbindbar und daran positionierbar. Beim Ausführungsbeispiel ist hierzu eine Axialkupplung 22 und eine Drehpositionskupplung 23 vorgesehen. Die Axialkupplung 22 ist durch eine Kupplungsausnehmung 22a im Endbereich des Halteschaftes 6b und einen Kupplungszapfen 22b gebildet, der in einer radialen Ausnehmung in der Aufnahmehülse 12 radial verschiebbar gelagert ist, durch die Kraft einer Feder 24 in seine in die Kupplungsausnehmung 22a einfassende Kupplungsstellung vorgespannt ist und durch einen Druckschieber 25 radial entkuppelbar, der auf der dem Werkzeug 6 abgewandten Seite gegen die Kraft einer Feder 26 durch einen Fingerdruck einschiebbar gelagert ist.

Die Drehpositionskupplung 22 weist wenigstens eine Kupplungsausnehmung 23a und wenigstens einen Kupplungszapfen 23b auf, die an der Aufnahmehülse 12 und dem Halteelement 6b angeordnet sind und in der eingesteckten Position des Halteelements 6b formschlüssig ineinandergreifen. Es sind wenigstens zwei Paare Kupplungsausnehmungen 22a und Kupplungszapfen 22b vorgesehen, die in der Umfangsrichtung winkelversetzt, z.B. einander diametral gegenüberliegend, angeordnet sind, so dass der Halteschaft 6b in zwei in der Umfangsrichtung winkelversetzt zueinander angeordneten Arbeitsstellungen kuppelbar ist. Beim Ausführungsbeispiel ist die Drehpositionierkupplung 23 durch eine Mehrzahl auf dem Umfang gleichmäßig verteilt angeordnete Kupplungsausnehmungen 23a und ein oder mehrere entsprechend gleichmäßig verteilte Kupplungszapfen 23b gebildet, wobei der oder die Kupplungszapfen 23b vorzugsweise an der Aufnahmehülse 12 und die wenigstens eine Kupplungsausnehmung 23a am Halteschaft 6b angeordnet sind, insbesondere an der nahe der Einstecköffnung 12a. Die wenigstens eine Kupplungsausnehmung 23a ist axial offen, so dass die Drehpositionierkupplung 23 durch eine axiale Bewegung in die Kupplungsstellung bringbar ist. Die Drehpositionierkupplung 23 ist vorzugsweise eine Vielkeilverbindung.

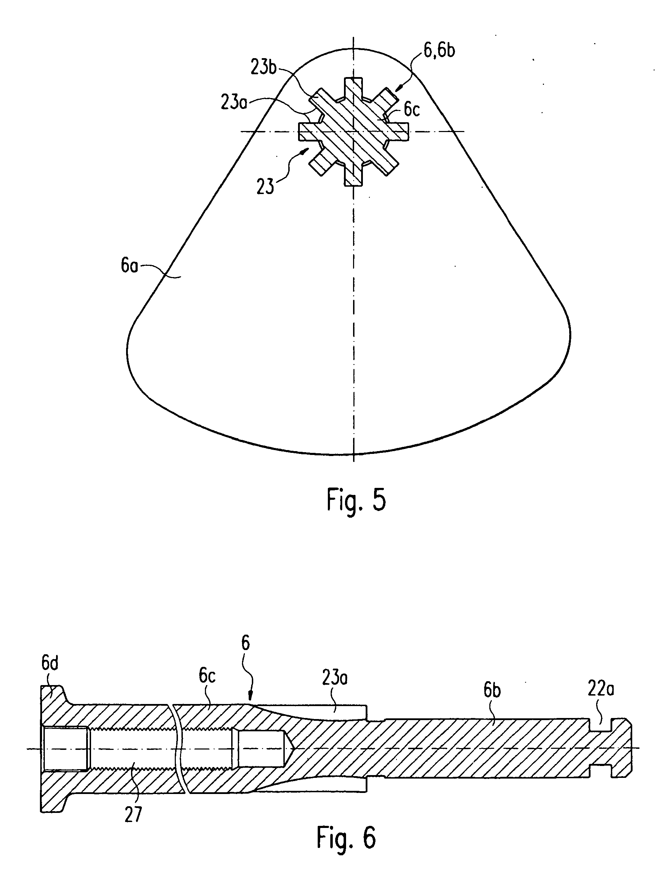

Wie insbesondere Fig. 5 zeigt, ist die Kupplungsausnehmung 22a durch eine Ringnut gebildet, die ein Kuppeln des Halteschaftes 6b in einer wahlweisen Drehposition der Drehpositionierkupplung 22 ermöglicht. Die wenigstens eine Kupplungsausnehmung 22a ist in einem bezüglich dem Halteschaft 6b verdickten Schaftabschnitt 6c angeordnet und sie läuft in der dadurch gebildeten Schaftstufe axial aus.

Beim Ausführungsbeispiel ist der Arbeitsabschnitt 6a eine vom Halteschaft einseitig abstehende Segmentscheibe, die einteilig mit dem Halteschaft 6b ausgebildet sein kann oder mit diesem lösbar verbunden sein kann, z.B. durch eine Befestigungsschraube, die die Segmentscheibe in einem Loch durchsetzt und in eine Gewindebohrung 27 im zugehörigen Endbereich des Halteschaftes 6b einschraubbar ist. Um dem an der ebenen Stirnseite des Halteelements bzw. Halteschaftes 6b anliegenden Arbeitsabschnitt 6a eine größere Anlagefläche zu verwirklichen, ist das Halteelement an diesem Ende durch einen Ringflansch 6d im Querschnitt vergrößert.

Das Handstück 3 weist in seinem vorderen Endbereich eine Auslassdüse 31 für ein ihr zuführbares strömendes Medium auf, die auf das Werkzeug 6, insbesondere auf den Arbeitsabschnitt 6a, oder auf die mit diesem behandelbare Arbeitsstelle gerichtet ist. Die Auslassdüse 31 ist in einem axialen Abstand von der Haltevorrichtung 5 bzw. der Aufnahmehülse 12 auf der Werkzeugseite des Handstücks 3 angeordnet, wobei sie vorzugsweise nach hinten versetzt ist und ihre Düsenachse 31a nach vorne divergent verläuft und einen spitzen Winkel W1 mit der Längsachse 13 des Handstücks 3 einschließt, der vorzugsweise etwa 50° bis 80°, insbesondere etwa 60° bis 70°, beträgt. Um die Auslassdüse 31 auf unterschiedliche Stellen des Werkzeugs 6 oder der Behandlungsstelle oder auf unterschiedliche Stellungen des Werkzeuges 6 bzw. seines Arbeitsabschnitts 6a richten zu können, ist sie quer zu ihrer Düsenachse 31a hin und her bewegbar, insbesondere hin und her schwenkbar. Zur Führung der Auslassdüse 31 in einer vorbestimmten Bahn ist vorzugsweise eine Führung 32 vorgesehen, insbesondere eine Schwenkführung, deren Schwenkachse sich insbesondere in der Längsrichtung des Handstücks 3 erstreckt und beim Ausführungsbeispiel koaxial zur Längsmittelachse 13 und Drehachse der Antriebswelle 18 gerichtet ist. Dabei ist die Auslassdüse 31 auf der Werkzeugseite des Handstücks 3 angeordnet auf der sich die Einstecköffnung 12a befindet, wobei sie einen in der Längsrichtung des Handstücks 3 gerichteten Abstand vom Werkzeug 6 aufweist bzw. einen längs gerichteten Abstand von der Drehachse 12b der Aufnahmehülse 12, der beim Ausführungsbeispiel nach hinten gerichtet ist. Um trotz dieses Abstands auf die betreffenden Abschnitte des Werkzeugs 6 bzw. der Behandlungsstelle gerichtet zu sein, schließt die Auslassdüse 31 mit der Längsachse des Handstücks 3 einen spitzen Winkel W ein.

Bei dem durch die Auslassdüse 31 zuführbaren strömungsfähigen Medium handelt es sich vorzugsweise um Wasser oder Luft oder ein Wasser/Luft-Gemisch (Spray). Die Zuführung des Mediums zu der Auslassdüse 31 kann intern durch das Handstück 3 oder extern erfolgen. Im letzteren Fall ist eine in der Umfangsrichtung versetzte oder vorzugsweise auf der der Auslassdüse 31 gegenüberliegenden Seite angeordnete Zuführungsleitung 33 vorgesehen, von der nur ein Anschlussrohr 34 dargestellt ist, das mit einem radialen oder schrägen Schenkel an das Handstück 3 angeschlossen ist, z.B. in ein Loch eingeklebt oder eingelötet ist und einen sich nach hinten erstreckenden Anschlussschenkel aufweist. Das Anschlussrohr 34 besteht vorzugsweise aus Metall. Ein zugehöriger Zuführungsleitungsschlauch kann auf das Anschlussrohr 34 dicht aufgeschoben werden.

Die Auslassdüse 31 kann aus einem sich vorzugsweise gerade erstreckenden Düsenrohr 35 bestehen, dass in einem Loch des Handstücks 3 dicht eingesetzt und befestigt ist, z.B. durch Kleben oder Löten. Ein der Auslassdüse 31 vorgeordneter Längsabschnitt 33a der Zuführungsleitung 33 ist vorzugsweise durch einen Ringkanal 36 gebildet, der beim Ausführungsbeispiel durch eine Ringnut in einem Drehlagerzapfen 37a des Handstücks 3 angeordnet ist, der von einer auf ihm drehbar gelagerten Drehhülse 38 umgeben ist, an der die Auslassdüse 31 ausgehend vom Ringkanal 36 angeordnet ist und vorzugsweise auch das Düsenrohr 35 angeordnet und mit dem Ringkanal 36 verbunden ist. Hierdurch ist nicht nur eine einfache Ausgestaltung für eine Durchquerung des Handstücks mit der Zuführungsleitung 33 gebildet, sondern auch ein die Führung 32 bildendes Drehlager 37, in dem die Auslassdüse 31 seitlich hin und her schwenkbar ist.

Um die Handhabbarkeit beim Bewegen und Einstellen der Auslassdüse 31 auf die gewünschte Stelle zu vereinfachen, ist es vorteilhaft, die Bewegungslänge durch wenigstens einen Anschlag 41 zu begrenzen, so dass die Bedienungsperson die Auslassdüse 31 manuell lediglich gegen den Anschlag 41 zu bewegen braucht, und die Auslassdüse sich dann in ihrer richtigen und gewünschten Position befindet. Beim Ausführungsbeispiel, bei dem die Auslassdüse 31 aus einer etwa mittigen Stellung in einander gegenüberliegende Richtungen bewegbar ist, sind zwei Anschläge 41 gebildet, die die Bewegungen der Auslassdüse 31 aus ihrer Mittelstellung heraus in die einander gegenüberliegenden Bewegungsrichtungen begrenzen. Bei einer vorliegenden Schwenkbewegung kann der Schwenkwinkel W2 aus der Mittelstellung etwa 10° bis 20°, insbesondere etwa 15° betragen.

Beim vorliegenden Ausführungsbeispiel sind die Anschläge 41 durch eine Längsnut 42 und einen darin angeordneten Anschlagzapfen 43 gebildet, wobei die Enden der Längsnut 42 die Anschläge 41 bilden. Die Längsnut 42 ist vorzugsweise in der Drehhülse 38 angeordnet, und der Anschlagzapfen 43 sitzt im Drehlagerzapfen 37a und er ragt radial nach außen in das Langloch 42 hinein.

Der den Ringkanal 36 bildende Zuführungsleitungsabschnitt ist auf beiden Seiten der Auslassdüse 31 durch Dichtelemente 44 abgedichtet, die in Aufnahmenuten 45 sitzen und beim Ausführungsbeispiel durch Dichtungsringe, insbesondere O-Ringe, und Ringnuten zwischen der Drehhülse 38 und dem Drehlagerzapfen 37 gebildet sind. Vorzugsweise sind die Aufnahmenuten 45 im Drehlagerzapfen 37 angeordnet.

Beim Ausführungsbeispiel ist der vordere Endbereich des Handstücks 3 ein sog. Handstück-Kopfstück 3a mit einem verdickten Kopf, das einen rückseitigen Lagerzapfen 47 aufweist, mit dem er in eine entsprechende Aufnahmebohrung eines hinteren Längsabschnitts des Handstücks 3 einsteckbar und fixierbar ist.

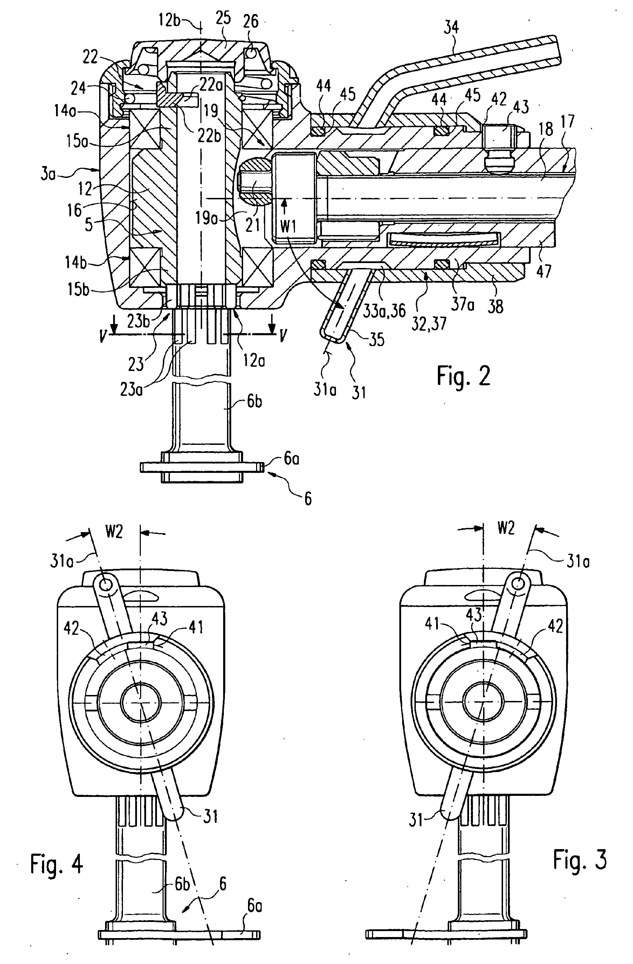

Die Drehpositionierkupplung 23 ermöglicht es, den einseitig abstehenden Arbeitsabschnitt 6a in unterschiedlichen Drehstellungen zu montieren, z.B. in die einander gegenüberliegenden seitlichen Stellungen gemäß Fig. 2 und 3. Um diese Stellungen mit dem Mediumstrahl beaufschlagen zu können, wird die Auslassdüse 31 jeweils in die gewünschte Stellung bewegt, in der sie auf die gewünschte Stelle am Werkzeug 6 oder in der Behandlungszone gerichtet ist und am zugehörigen Anschlag 41 bewegungsbegrenzt ist.

Die Erfindung ist nicht auf einen Werkzeugantrieb eingeschränkt, der das Werkzeug 6 hin und her schwenkt. Beim Werkzeug 6 kann es sich z.B. auch um eine Scheibe handeln, die auch rotierend antreibbar sein könnte und auf mehreren oder allen Seiten einen Arbeitsabschnitt 6a aufweisen kann. Auch bei einem solchen Werkzeug lässt sich die Auslassdüse 31 jeweils auf die gewünschte Arbeitszone einstellen.

Bei beiden in Fig. 1 mit sichtbaren Linien und mit Strichpunkt-Linien dargestellten Ausführungsbeispielen handelt es sich bei dem Handstück um ein sogenanntes "Winkel-Handstück" . Das Winkel-Handstück, dessen angedeuteter Handgriff sich gerade erstreckt, eignet sich für eine medizinische oder dentalmedizinische Behandlung. Bei dem mit sichtbaren Linien dargestellten Winkel-Handstück mit einem Kontrawinkel W3 handelt es sich um ein typisches dentalmedizinisches Handstück.

## Patentansprüche

1. Medizinisches, insbesondere dentalmedizinisches, längliches Handstück (3) mit einer Haltevorrichtung (5) für ein Werkzeug (6), die im vorderen Endbereich des Handstücks (3) an einer Werkzeugseite des Handstücks (3) eine Einstecköffnung (12a) für das Werkzeug (6) aufweist,
wobei im vorderen Endbereich des Handstücks (3) an dessen Werkzeugseite eine Auslassdüse (31) für ein strömendes Medium angeordnet ist,
wobei die Auslassdüse (31) zwecks Einstellung der Richtung des Medienstrahles verstellbar ist,
**dadurch gekennzeichnet,**
**dass** die Auslassdüse (31) in einer Führung (32) in der Umfangsrichtung des Handstücks (3) bewegbar ist.

2. Handstück nach Anspruch 1,
**dadurch gekennzeichnet,**
**dass** die Auslassdüse (31) hin und her schwenkbar ist.

3. Handstück nach Anspruch 1 oder 2,
**dadurch gekennzeichnet,**
**dass** die Auslassdüse (31) aus einer Mittelstellung heraus in zwei einander gegenüberliegende Bewegungsrichtungen bewegbar ist.

4. Handstück nach einem der vorherigen Ansprüche,
**dadurch gekennzeichnet,**
**dass** die Auslassdüse (31) einen in der Längsrichtung des Handstücks (3) gerichteten Abstand von der Einstecköffnung (12a) aufweist, der vorzugsweise nach hinten gerichtet ist.

5. Handstück nach Anspruch 3,
**dadurch gekennzeichnet,**
**dass** die Auslassdüse (31) an einer Drehhülse (38) angeordnet ist, die im Umfangsbereich des Handstücks (3) drehbar gelagert ist.

6. Handstück nach Anspruch 5,
**dadurch gekennzeichnet,**
**dass** zwischen der Drehhülse (38) und einem sie tragenden Drehlagerzapfen (37) eine Ringnut (36) angeordnet ist, die mit der Auslassdüse (31) verbunden ist.

7. Handstück nach Anspruch 6,
**dadurch gekennzeichnet,**
**dass** das Handstück (3) bezüglich der Auslassdüse (31) in der Umfangsrichtung versetzt ein Anschlusselement (34) für eine externe Medium-Zuführungsleitung (33) aufweist.

8. Handstück nach Anspruch 6 oder 7,
**dadurch gekennzeichnet,**
**dass** die Auslassdüse (31) auf beiden Seiten der Ringnut (36) durch Dichtelemente abgedichtet ist.

9. Handstück nach Anspruch 8,
**dadurch gekennzeichnet,**
**dass** die Dichtelemente jeweils durch einen in einer Ringnut (45) angeordneten Dichtring, insbesondere O-Ring, gebildet ist.

10. Handstück nach einem der vorherigen Ansprüche,
**dadurch gekennzeichnet,**
**dass** der Bewegungsweg der Auslassdüse (31) durch einen oder zwei Anschläge (41) begrenzt ist.

11. Handstück nach Anspruch 10,
**dadurch gekennzeichnet,**
**dass** zur Anschlagbegrenzung ein Anschlagzapfen (43) vorgesehen ist, der in einer Längsnut (42) angeordnet ist.

12. Handstück nach Anspruch 11,
**dadurch gekennzeichnet,**
**dass** die Längsnut (42) in der Drehhülse (38) angeordnet ist.

13. Handstück nach einem der vorherigen Ansprüche,
**dadurch gekennzeichnet,**
**dass** zwischen der Haltevorrichtung (5) und dem Werkzeug (6) eine Drehpositionierkupplung (23) angeordnet ist, die ein Verbinden des Werkzeugs (6) in zwei oder mehr in die Umfangsrichtung versetzten, vorzugsweise gleichmäßig versetzten Stellungen ermöglicht.

14. Handstück nach Anspruch 13,
**dadurch gekennzeichnet,**
**dass** die Drehpositionierkupplung (23) im Bereich der Einstecköffnung (12a) angeordnet ist und vorzugsweise durch eine Vielkeilverbindung gebildet ist.

15. Handstück nach einem der vorherigen Ansprüche,
**dadurch gekennzeichnet,**
**dass** die Haltevorrichtung (5) eine Aufnahmehülse (12) aufweist, die in einer sich quer im Handstück (3) erstreckenden Stellung bewegbar gelagert ist.

16. Handstück nach einem der vorherigen Ansprüche,
**dadurch gekennzeichnet,**
**dass** die Haltevorrichtung (5) oder die Aufnahmehülse (12) durch einen Antrieb (17) hin und her schwenkbar ist.

17. Handstück nach einem der vorherigen Ansprüche,
**dadurch gekennzeichnet,**
**dass** das Werkzeug (6) einen Werkzeugschaft (6b) und einen sich vorzugsweise quer dazu erstreckenden Arbeitsabschnitt (6a) aufweist.

18. Handstück nach Anspruch 17,
**dadurch gekennzeichnet,**
**dass** der Arbeitsabschnitt (6a) durch eine Scheibe gebildet ist, die vorzugsweise einseitig angeordnet ist und insbesondere durch eine Segmentscheibe gebildet ist.

## Claims

1. Medical, in particular dental-medical, elongate handpiece (3) having a holder device (5) for a tool (6), which has in the forward end region of the handpiece (3), on a tool side of the handpiece (3), an insertion opening (12a) for the tool (6),
wherein in the forward end region of the handpiece (3), on the tool side thereof, there is arranged an outlet nozzle (31) for a flowing medium,
wherein for setting the direction of the jet of medium the outlet nozzle (31) is adjustable,
**characterised in that**,
the outlet nozzle (31) is movable in a guide (32) in the circumferential direction of the handpiece (3).

2. Handpiece according to claim 1,
**characterised in that**,
the outlet nozzle (31) is pivotable back and forth.

3. Handpiece according to claim 1 or 2,
**characterised in that**,
the outlet nozzle (31) is movable out of a middle position in two mutually opposite directions of motion.

4. Handpiece according to any preceding claim,
**characterised in that**,
the outlet nozzle (31) has a spacing, directed in the longitudinal direction of the handpiece (3), from the insertion opening (12a), which is preferably directed rearwardly.

5. Handpiece according to claim 3,
**characterised in that**,
the outlet nozzle (31) is arranged on a rotary sleeve (38) which is rotatably mounted in the circumferential region of the handpiece (3).

6. Handpiece according to claim 5,
**characterised in that**,
a ring groove (36) which is connected to the outlet nozzle (31) is arranged between the rotary sleeve (38) and a rotary bearing pin (37) carrying the sleeve.

7. Handpiece according to claim 6,
**characterised in that**,
the handpiece (3) has a connection element (34), offset in the circumferential direction with respect to the outlet nozzle (31), for an external medium delivery line (33).

8. Handpiece according to claim 6 or 7,
**characterised in that**,
the outlet nozzle (31) is sealed off by sealing elements on the two sides of the ring groove (36).

9. Handpiece according to claim 8,
**characterised in that**,
the sealing elements are in each case formed by a sealing ring, in particular 0-ring, arranged in a ring groove (45).

10. Handpiece according to any preceding claim,
**characterised in that**,
the movement path of the outlet nozzle (31) is limited by one or two stops (41).

11. Handpiece according to claim 10,
**characterised in that**,
for stop limitation there is provided a stop pin (43) which is arranged in a longitudinal groove (42).

12. Handpiece according to claim 11,
**characterised in that**,
the longitudinal groove (42) is arranged in the rotary sleeve (38).

13. Handpiece according to any preceding claim,
**characterised in that**,
a rotary positioning coupling (23) is arranged between the holding device (5) and the tool (6) which makes possible a connection of the tool (6) in two or more positions offset in the circumferential direction, preferably uniformly offset.

14. Handpiece according to claim 13,
**characterised in that**,
the rotary positioning coupling (23) is arranged in the region of the insertion opening (12a) and is preferably formed by means of a multiple wedge connection.

15. Handpiece according to any preceding claim,
**characterised in that**,
the holding device (5) has a receiving sleeve (12) which is mounted movably in a position extending transversely in the handpiece (3).

16. Handpiece according to any preceding claim,
**characterised in that**,
the holding device (5) or the receiving sleeve (12) is pivotable back and forth by means of a drive (17).

17. Handpiece according to any preceding claim,
**characterised in that**,
the tool (6) has a tool shaft (6b) and a working section (6a) preferably extending transversely thereto.

18. Handpiece according to claim 17,
**characterised in that**,
the working section (6a) is formed by a disk which is arranged preferably one-sided and is in particular formed by a segment disk.

## Revendications

1. Pièce à main (3) allongée, médicale, en particulier de médecine dentaire, comprenant un dispositif de retenue (5) pour un outil (6), qui présente dans la zone d'extrémité avant de la pièce à main (3) sur un côté outil de la pièce à main (3) une ouverture d'introduction (12a) pour l'outil (6),
une buse d'évacuation (31) pour un fluide circulant étant disposée dans la zone d'extrémité avant de la pièce à main (3) sur son côté outil,
la buse d'évacuation (31) étant réglable pour le réglage de la direction du jet de fluide,
**caractérisée en ce que**
la buse d'évacuation (31) peut être déplacée dans un guide (32) dans le sens périphérique de la pièce à main (3).

2. Pièce à main selon la revendication 1,
**caractérisée en ce que**
la buse d'évacuation (31) peut être basculée d'un côté ou de l'autre.

3. Pièce à main selon la revendication 1 ou 2,
**caractérisée en ce que**
la buse d'évacuation (31) peut être déplacée à partir d'une position centrale dans deux directions de déplacement se faisant face.

4. Pièce à main selon l'une quelconque des revendications précédentes,
**caractérisée en ce que**
la buse d'évacuation (31) présente par rapport à l'ouverture d'introduction (12a) une distance dirigée dans la direction longitudinale de la pièce à main (3) qui est orientée de préférence vers l'arrière.

5. Pièce à main selon la revendication 3,
**caractérisée en ce que**
la buse d'évacuation (31) est disposée sur une douille rotative (38) qui est logée de façon rotative dans la zone périphérique de la pièce à main (3).

6. Pièce à main selon la revendication 5,
**caractérisée en ce que**
une rainure annulaire (36), qui est reliée à la buse d'évacuation (31), est disposée entre la douille rotative (38) et un tenon à palier rotatif (37) qui la porte.

7. Pièce à main selon la revendication 6,
**caractérisée en ce que**
la pièce à main (3) présente par rapport à la buse d'évacuation (31), dans le sens périphérique, un élément de raccordement (34) pour une conduite externe d'alimentation en fluide (33).

8. Pièce à main selon la revendication 6 ou 7,
**caractérisée en ce que**
la buse d'évacuation (31) est rendue étanche des deux côtés de la rainure annulaire (36) par des éléments d'étanchéité.

9. Pièce à main selon la revendication 8,
**caractérisée en ce que**
les éléments d'étanchéité sont formés respectivement par une bague d'étanchéité, en particulier un joint torique, disposé dans une rainure annulaire (45).

10. Pièce à main selon l'une quelconque des revendications précédentes,
**caractérisée en ce que**
la course de déplacement de la buse d'évacuation (31) est limitée par une ou deux butées (41).

11. Pièce à main selon la revendication (10),
**caractérisée en ce que**
il est prévu pour la limitation de butée un tenon de butée (43) qui est disposé dans une rainure longitudinale (42).

12. Pièce à main selon la revendication 11,
**caractérisée en ce que**
la rainure longitudinale (42) est disposée dans la douille rotative (38).

13. Pièce à main selon l'une quelconque des revendications précédentes,
**caractérisée en ce que**
entre le dispositif de retenue (5) et l'outil (6) est disposé un accouplement de positionnement en rotation (23) qui permet une liaison de l'outil (6) dans deux ou plus de deux positions décalées dans le sens périphériques, de préférence décalées de façon uniforme.

14. Pièce à main selon la revendication 13,
**caractérisée en ce que**
l'accouplement de positionnement en rotation (23) est disposé dans la zone de l'ouverture d'introduction (12a) et est formé de préférence par une liaison cannelée à cales multiples.

15. Pièce à main selon l'une quelconque des revendications précédentes,
**caractérisée en ce que**
le dispositif de retenue (5) présente une douille de logement (12) qui est montée de façon mobile dans une position s'étendant transversalement dans la pièce à main (3).

16. Pièce à main selon l'une quelconque des revendications précédentes,
**caractérisée en ce que**
le dispositif de retenue (5) ou la douille de logement (12) peut être basculée d'un côté ou de l'autre par un entraînement (17).

17. Pièce à main selon l'une quelconque des revendications précédentes,
**caractérisée en ce que**
l'outil (6) présente une tige d'outil (6b) et une partie de travail (6a) s'étendant de préférence transversalement à la tige.

18. Pièce à main selon la revendication 17,
**caractérisée en ce que**
la partie de travail (6a) est formée par un disque qui est disposé de préférence sur un côté et est formé en particulier par un disque à segment.
